(19)

Europäisches
Patentamt
European
Patent Office
Office européen
des brevets

(11) **EP 3 708 158 A1**

(12) **EUROPEAN PATENT APPLICATION**

(43) Date of publication:
**16.09.2020 Bulletin 2020/38**

(51) Int Cl.:
***A61K 31/167*** [(2006.01)]   ***A61P 7/06*** [(2006.01)]
***A61P 43/00*** [(2006.01)]

(21) Application number: **19161933.7**

(22) Date of filing: **11.03.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Hartis-Pharma SA
1260 Nyon (CH)**

(72) Inventors:
- **NIESOR, Joseph Eric
  1260 NYON (CH)**
- **BENGHOZI, Renée
  94220 CHARENTON LE PONT (FR)**
- **LAMOUR, François
  68730 BLOTZHEIM (FR)**

(74) Representative: **reuteler & cie SA
Chemin de la Vuarpillière 29
1260 Nyon (CH)**

(54) **ALLOSTERIC REGULATORS OF HEMOGLOBIN USEFUL IN THE TREATMENT OF SICKLE
CELL TRAIT**

(57)   The present invention is related to dalcetrapib or dalcetrapib-thiol, or a combination thereof for use in the prevention and/or treatment of disorders characterized by inefficient transport and delivery of oxygen to peripheral tissues, in particular sickle cell trait and thalassemia. The invention in particular relates to pharmaceutical formulations, regimens, methods of treatment and uses thereof.

EP 3 708 158 A1

SEQUENCE LISTING

<110> HARTIS_PHARMA SA

<120> ALLOSTERIC REGULATORS OF HEMOGLOBIN USEFUL IN THE TREATMENT OF SICKLE CELL TRAIT

<130> P2313EP00

<160> 1

<170> PatentIn version 3.5

<210> 1
<211> 22
<212> PRT
<213> Artificial Sequence

<220>
<223> human hemoglobin synthetic peptide containing Cys93

<400> 1

Gly Thr Phe Ala Thr Leu Ser Glu Leu His Cys Asp Lys Leu His Val
1               5                   10                  15

Asp Pro Glu Asn Phe Arg
            20

**Description**

**Field of the Invention**

The present invention relates to the use of dalcetrapib in the prevention and/or treatment of disorders characterized by an inefficient transport and delivery of oxygen to peripheral tissues, in particular pathological complications of Sickle cell trait and of Thalassemia.

**Background of the Invention**

The human erythrocyte hemoglobin molecule is composed of two $\alpha$-chains ($\alpha$1 and $\alpha$2) and 2 $\beta$-chains ($\beta$1 and $\beta$2) forming a heterotetramer. Human hemoglobin functions as an oxygen carrier that transports oxygen from the lungs to the other peripheral tissues of the body. Each of the four hemoglobin units contains a heme group that is capable of binding oxygen or other ligands.

The capacity of hemoglobin to transport and deliver oxgygen is allosterically regulated. The allosteric nature of hemoglobin function is the result of the subunits packing in the tetramer that allows significant quaternary structure changes to occur during ligand binding. The tetramer assembles forming two different interfaces. In contrast to the $\alpha$1$\beta$2/$\alpha$2$\beta$1-interfaces that mediate the quaternary changes associated with ligand binding (Ackers et al., 1992, Science, 255(5040):54-63) the $\alpha$1$\beta$1 and $\alpha$2$\beta$2 interface of the dimer is quite stable with the relative positions of the two subunits being unmodified by the state of ligation of the protein. There are six cysteine residues in hemoglobin. The $\beta$-subunit has two cysteine residues, $\beta$93Cys and $\beta$112Cys, that are associated with the two hemoglobin interfaces and $\beta$93Cys is located at the $\alpha$1$\beta$2/$\alpha$1$\beta$2-interface.

Khan et al. 2001, Biochemistry, 40, 7581-7592 indicated that Cys $\beta$93 is situated in a conformationally plastic domain containing residues whose interactions are directly linked to allosteric properties of the hemoglobin tetramer. The reactivity of the $\beta$93 sulfhydryl toward many reactants is highly sensitive to both the quaternary and tertiary conformation of hemoglobin. It has been proposed that *in vivo* the functional roles of $\beta$93 are also modulated by the overall protein structure. Recently, it has been suggested that $\beta$93Cys plays a role in nitric oxide (NO) transport and delivery (Jia et al., 1996, Nature. 380:221-226). NO is known to play important functional roles in a variety of physiological systems. Within the vasculature, NO induces vasodilation, inhibits platelet aggregation, prevents neutrophil/platelet adhesion to endothelial cells, inhibits smooth muscle cell proliferation and migration, regulates programmed cell death (apoptosis) and maintains endothelial cell barrier function.

Sickle cell trait (SCT) is a genetic trait characterized by the inheritance of a normal hemoglobin gene (HbA) from 1 parent and an abnormal, mutated hemoglobin gene, the sickle hemoglobin gene (HbS), from the other parent and therefore the carriers have both non mutated hemoglobin (HbA) and mutated hemoglobin HbS (AS genotype) in their Red Blood Cells (RBC).

In contrast to Sickle Cell Disease (SCD), where both hemoglobin alleles are mutated, only 1 abnormal hemoglobin allele of the hemoglobin gene is inherited in SCT. Allison, 1954, Br. Med. J., 1(4587): 290-294 proposed that SCT protects against malarial infection which explains the high incidence of SCT in region where malaria is endemic. It is estimated that 300 million people worldwide are heterozygous for the HbS mutation as described above. Although 40-50% of the RBC hemoglobin contains the mutation in SCT, polymerization of Hb as observed in SCD can occur in the presence of low $O_2$ partial pressure. For several decades SCT has been considered as benign with minor or no complications related to sickling and no clear and life threatening phenotype like SCD. However as early as 1963, the vulnerability of the SCT subjects to certain stressful conditions such as high altitude and infection was observed (Thompson, 1963, Br. Med. J., 14;2(5371):1534) and is now established beyond doubt. Recently, Lu et al., 2018, Am. J. Hematol., 93:1227-1235, showed that even with its lower sickle hemoglobin concentration, sickle trait blood apparent viscosity increases with decreasing oxygen tension and may stop flowing under completely anoxic conditions. Indeed, John, 2010, OMJ. 25, 3-8, noted that numerous complications and risks are associated with SCT such as:

a) Splenic infarction under the condition of i) high altitude, ii)exercise, b) hypoxemia, isothenuria with loss of maximal renal concentrating ability, c) hematuria secondary to renal papillary necrosis, d) fatal exertional heat illness with exercise, e) sudden idiopathic death with exercise, f) glaucoma or recurrent hyphema following a first episode of hyphema, g) bacteruria in women, h) bacteruria or pyelonephritis associated with pregnancy, i) renal medullary carcinoma in young people (ages 11 to 39 years), j) early onset of end stage renal disease from autosomal dominant polycystic kidney. Naik et al. 2014, JAMA, 312(20):2115-2125 confirmed in a cohort study that, among African Americans, the presence of SCT was associated with an increased risk of Chronic Kidney Disease (CKD), decline in estimated gglomerular Filtration Rate (eGFR), and albuminuria, compared with non-carriers. More importantly, numerous case studies have reported exercise-related death in subjects with SCT over the past 5 decades and renal abnormalities are among the best-recognized complications of SCT. SCT was also reported as associated with a significantly higher risk of exertional rhabdomyolysis Nelson and Folsom (Nelson et al., 2016, N. Engl. J. Med., 4; 375(5): 435-442; Folsom et al. 2015, J Thromb Haemost., 13(1): 2-9) observed that African Americans with SCT carries a 2-fold increased risk of pulmonary embolism. In addition D-dimer levels are significantly higher among SCT subjects (Naik et al., 2016, Blood, 18,127, 2661-2663) carriers compared with non-carriers. Moreover, it has been conclusively demonstrated that individuals with SCT have impaired urinary concentrating ability (reviewed by Key et al., 2015, Br. J. Haematol., 170(1): 5-14). Very recently, Skinner at al., 2018, Diabetes Care, 41:1-8 showed that SCT subjects who have Type 2 diabetes (T2D) have higher incidence of retinopathy, nephropathy, and hypertension than diabetics or SCT alone and suggested that SCT could exacerbate multiple factors that increase the risk of developing T2D- related vascular complications including arterial stiffness, blood hyperviscosity, Advanced Glycation End products accumulation, and increased E-selectin expression.

Therefore, due to specific complications of chronic diseases when associated with SCT, this clinical condition corresponds to pathological and clinical profiles clearly different from SCD. These clinical observations are very likely due to the intrinsic lower capacity of HbS (even more in monozygotes) to transport and deliver oxygen; further exacerbated in pathologic clinical situation such as diabetes, alzheimer' s disease and vascular diseases in general.

Alpha- and β-thalassemia, result from the defective synthesis of the α and β-globin chains of hemoglobin, respectively, are recognized as the most common monogenic diseases in humans (Weatherall et al., 1996, Nat Med,2:847-9; Strader et al., 2016, Redox Biology,8, 363-374) showed that for the first time that unpaired α globin is oxidatively unstable Their work also indicates that α subunit oxidative side reactions have a more profound impact on the β subunit; specifically irreversible oxidation of βCys93 in the presence of $H_2O_2$ which ultimately leads to β subunit unfolding and heme loss. Heme loss is a major trigger of oxidative stress in thalassemia.

Although targeting Cys93 of the β-chain of Hb for allosterically improving hemoglobin capacity to carry and deliver oxygen to tissues has been considered (Kassa et al., 2017, Metallomics, 9(9):1260-1270), the search for clinically useful compounds acting on this target have failed so far. Allosteric modulators of hemoglobin have been described as nitric oxide releasing moieties in US 9,765,017 and a method for improving the oxygen releasing ability of hemoglobin to organs and peripheral tissues was described in US 10, 047,063. US 9,713,602 describes phthalide compounds to increase the oxygen release efficiency of a hemoglobin-based blood substitute.

More importantly, no compound with high affinity binding for Cys93 and acceptable biovalaibility have been discovered. The safety and unfavorable bioavailabilty of SH containing compound able to interact with Cys93 has precluded so far the entry of such compound into preclinical toxicology and pharmacological studies. In addition, the focus has been until now around vanillin derivatives which form a Shiff base with Valine 1 (Metcalf et al., 2017, ACS Med. Chem. Lett., 8, 321-326) to allosterically affect Hb function. These compounds are unlikely to be active in patients with high level of hemoglobin glycation such as diabetes since Val1 of Hb is a major site of non specific Hb Glycation.

Considering the absence of a life threatening phenotype in SCT, the prevention of diseases risks and complications associated with SCT requires drugs specifically designed to treat these chronic clinical conditions similar to antihypertensives, lipid lowering drugs, oral antidiabetics etc. which are used to treat, in most cases, asymptomatic patients. Oral bioavailability, low dose, satisfactory long term safety upon chronic treatment are key parameters not fulfilled by the current SCD treatments such as the mutagenic and genotoxic hydroxyurea. Thus, there is an urgent need for finding new compounds having the potential of interacting with Cys93 of hemoglobin and which could allosterically affect its properties in a favourable manner to treat various hemoglobinopathies, in particular SCT.

Dalcetrapib is a compound that has been originally developed for treating cardiovascular diseases (Okamoto et al., 2000, Nature, 406:203-7) by increasing plasma HDL cholesterol levels. Until now all compounds of this HDL increasing therapeutic class have failed showing benefit in large outcome clinical trials aiming at decreasing cardiovascular disease incidence. The effect of this compound on RBC physiology, Hb and specifically HbS function and polymerization has neither been considered nor tested.

## Summary of the invention

The present invention relates to the non-obvious finding that compounds such as dalcetrapib S-[2-[[1-(2-ethylbutyl)cyclohexanecarbonyl]amino]phenyl] 2-methylpropanethioate CAS number 211513-37-0 and dalcetrapib-thiol (S-{2-([[1-(2-ethylbutyl) cyclohexyl]carbonyl]amino)phenyl}2-thiol) bind to Cys93 of the hemoglogin β-chain through a SS covalent bond resulting in a unique orally bioavailable allosteric regulator of hemoglobin, increasing its affinity for oxygen. Therefore, it is believed that dalcetrapib and dalcetrapib-thiol would be useful in the treatment of diseases or disorders which are characterized by inefficient transport and delivery of oxygen to peripheral tissues, thereby increasing oxygen exchange, transport and tissue delivery of $O_2$ by hemoglobin.

The present invention is based on the finding that dalcetrapib-thiol is an unexpectedly potent allosteric modulator of hemoglobin having a high affinity and selective covalent binding to Cys93 of the hemoglobin β chain which improves hemoglobin function: transport and delivery of oxygen. This unique property suggests an exceptional potential of dalcetrapib for the treatment of disorders characterized by inefficient transport and delivery of oxygen to peripheral tissues, in particular those which would benefit from allosterically modification of the hemoglobin behaviour such as SCT, hemorrhagic shock, cerebral and cardiovascular diseases (including hypertension, stroke, coronary disease, heart failure, neurodegenerative diseases such as Alzheimer and Parkinson diseases, multiorgan diseases (diabetes, cancer, inflammation, aging), eye dideases (macular degeneration, cataract, retinal degeneration), kidney diseases, autoimmune diseases, pulmonary diseases (asthma, COPD) and sepsis.

According to one aspect, the invention provides a dalcetrapib compound selected from dalcetrapib, dalcetrapib-thiol and a combination thereof for use in the prevention and/or treatment of a disease or disorder characterized by inefficient transport and delivery of oxygen to peripheral tissues, in particular for the prevention and/or treatment of SCT, such as diabetic complications in SCT subjects (such as retinopathy, kidney disease, hypertension), neurodegenerative diseases such as Alzheimer and Parkinson diseases, 2,3 biphosphoglycerate metabolic disorders, hypoxic tumors, acute or chronic ischemia, hemorrhagic shock, anemia, cerebral and cardiovascular diseases (including hypertension, stroke, coronary artery disease, heart failure and myocardial infarction), pulmonary diseases in particular chronic pulmonary diseases (e.g. asthma, Chronic Obstructive Pulmonary Disease -COPD), chronic hypoxia, altitude sickness, gangrene, anaerobic infections, eye diseases (e.g. macular degeneration, cataract, retinal degeneration), kidney diseases, autoimmune diseases and sepsis. According to a particular aspect, diabetic complications in thalassemic patients may also benefit form dalcetrapib treatment.

According to another aspect, the invention provides a pharmaceutical composition comprising a dalcetrapib compound selected from dalcetrapib, dalcetrapib-thiol and a combination thereof and at least a co-agent such as for example a further agent for improving the oxygen releasing ability of hemoglobin to organs and peripheral tissues such as an allosteric modulator of hemoglobin.

According to another aspect, the invention provides a use of a dalcetrapib compound selected from dalcetrapib, dalcetrapib-thiol and a combination thereof in the prevention and/or treatment of a disease or disorder characterized inefficient transport and delivery of oxygen to peripheral tissues, in particular for the prevention and/or treatment of SCT, such as diabetic complications in SCT subjects (such as retinopathy, kidney disease, hypertension), neurodegenerative diseases such as Alzheimer and Parkinson diseases, 2,3 biphosphoglycerate metabolic disorders, hypoxic tumors, acute or chronic ischemia, hemorrhagic shock, anemia, cerebral and cardiovascular diseases (including hypertension, stroke, coronary artery disease, heart failure and myocardial infarction), pulmonary diseases in particular chronic pulmonary diseases (e.g. asthma, COPD), chronic hypoxia, altitude sickness, gangrene, anaerobic infections, eye diseases (macular degeneration, cataract, retinal degeneration), kidney diseases, autoimmune diseases and sepsis.

According to another aspect, the invention provides a method of preventing and/or treating a disease or disorder characterized by inefficient transport and delivery of oxygen to peripheral tissues, said method comprising administering a dalcetrapib compound selected from dalcetrapib, dalcetrapib-thiol and a combination thereof, to a subject in need thereof.

*Detailed description*

As used herein, the expression "inefficient transport and delivery of oxygen to peripheral tissues" includes cellular and tissue damages due to low oxygen delivery to peripheral tissues through the microvasculature such as vascular complications (e.g. retinopathy, stroke, peripheral arterial disease, coronary artery disease) observed in diabetic complications in SCT subjects (such as retinopathy, kidney disease, hypertension), Alzheimer's disease, 2,3 biphosphoglycerate metabolic disorders, hypoxic tumors, acute or chronic ischemia, anemia, myocardial infarction, chronic pulmonary disease, chronic hypoxia, altitude sickness, gangrene and anaerobic infections. Subjects suffering from an efficient transport and delivery of oxygen to peripheral tissues present abnormal oxygen dissociation properties from Hb of red blood cells, for example, such dissociation characteristics could be assessed and measured as described in Guarnone et al., 1995, Haematologica, 80:426-30.

Subjects suffering from diabetic complications associated with SCT can be identified by standard guidelines such as used by Petersmann, 2018, Exp Clin Endocrinol Diabetes, 126(7):406-410 and Harris,2000, Nutr Metab Cardiovasc Dis.,10(6):305-9 wherein hemoglobin mutation, in particular HbS heterozygosity can be identified. Sickle Cell Scan™ (Nguyen-Khoa, 2018, Ann Biol Clin., 76(4):416-42) or other assay such as in Moat, 2014, Clin Chem., 60(2):373-80 could be used for this purpose.

The term "kidney disease or disorder" includes diabetic nephropathy, glomerulopathies, acute and chronic renal failure and hemolytic uremic syndrom. In a particular embodiment, the term according to the invention includes chronic kidney diseases or disorders.

The term "eye diseases" includes cataract including diabetic cataract, re-opacification of the lens post cataract surgery, diabetic and other forms of retinopathies like Glaucoma and Aged-related Macular degeneration (AMD).

The term "neurodegenerative disease or disorder" comprises a disease or a state characterized by a central nervous system (CNS) degeneration or alteration, especially at the level of the neurons such as Alzheimer's disease, vascular dementia, and Parkinson's disease.

The term "pulmonary disease" comprises asthma and chronic obstructive pulmonary diseases (COPD).

As used herein, "treatment" and "treating" and the like generally mean obtaining a desired pharmacological and physiological effect. The effect may be prophylactic in terms of preventing or partially preventing a disease, symptom or condition and complications thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions and complications.

According to a particular aspect, the efficacy of a treatment or a use according to the invention can be monitored through specific biomarkers in particular the determination of oxygen dissociation curves before and after treatment using Lorrca TM functional RBC analysis equipment from RR Mechatronics Manufacturing BV, Netherland as described by Rab et al., 2019, Am J Hematol., Feb 19 *in press* or the Hemox Analyzer (TCS Scientific) as described in *Guarnone et al., 1995, supra.*

The term "subject" as used herein refers to mammals. For examples, mammals contemplated by the present invention include humans and the like.

### *Mode of administration*

Compounds and compositions of this invention may be administered or delivered in any manner including, but not limited to, orally, parenterally, transdermally, intranasal or combinations thereof. Parenteral administration includes, but is not limited to, intravenous, intra-arterial, intra-peritoneal, subcutaneous and intramuscular.

In another particular embodiment, a compound according to the invention is administered systemically by injection.

According to a particular embodiment, a compound according to the invention can be administered at a dosage between 300 to 900 mg per day, in particular at a dosage of 600 mg per day.

Single oral doses of several grams may also be considered in specific pathological situations in which the bioavailability of dalcetrapib to plasma, RBC or Hb is impaired or when rapid onset of activity is required, typically from 1 g to about 5 g.

The dosage administered, as single or multiple doses, to an individual will vary depending upon a variety of factors, including pharmacokinetic properties, subject conditions and characteristics (gender, age, body weight, health, and size), extent of symptoms, concomitant treatments, frequency of treatment and the effect desired.

### *Combination*

According to one embodiment of the invention, the compounds according to the invention and pharmaceutical formulations thereof can be administered alone or in combination with a co-agent useful in the prevention and/or treatment of a disease.

According to a particular aspect, is provided a combination of at least one of dalcetrapib and dalcetrapib-thiol or a combination thereof with a co-agent selected from hydroxyurea, L-glutamine, a vanillin derivative, such as GBT-440 (2-hydroxy-6((2-(1-isopropyl-1Hpyrazol-5-yl) pyridin-3-yl)methoxy)benzaldehyde) and analogues thereof, an antidiabetic drug such as Biguanides, Sulfonylureas, Meglitinides, Thiazolidinediones, Alphaglucosidase inhibitors, Sodium-glucose co-transporter type 2 (SGLT2) inhibitors, Dipeptidyl peptidase-4 (DPPIV), inhibitor Glucagon-like peptide receptor agonists (GLP-1 RAs) and insulin and derivatives thereof, Amylin mimetics, a vasodilator such as Angiotensin Converting Enzyme (ACE) inhibitors, Angiotensin Receptor Blockers (ARBs), Calcium channel blockers, Alpha-adrenoreceptor antagonists (alpha blockers), Beta-2 adrenoreceptor agonists, Centrally acting sympatholytics, Endothelin receptor antagonists, Nitrates, Phosphodiesterase inhibitors, Potassium channel openers, Renin inhibitors, or a cardiovascular drug such as Antiplatelets, Anticoagulants, Beta- blockers, Diuretics, Cholesterol lowering agents, HDL raising drugs, Thrombolytics agents or a further agent for improving the oxygen releasing ability of hemoglobin to organs and peripheral tissues such as an allosteric modulator of hemoglobin such as those described in US 9,765,017 or agents described in US 10,047,063 or US 9,713,602.

According to a particular aspect, a co-agent is selected from vanillin and derivatives, GBT-440 and analogues thereof.

According to a further particular aspect, a combination of the invention is a a pharmaceutical composition comprising of at least one dalcetrapib compound selected from dalcetrapib, dalcetrapib-thiol and a mixture thereof and at least one co-agent of the invention or a mixture thereof and a pharmaceutically acceptable carrier, diluent or excipient thereof.

According to a particular aspect, the combination of the present invention is useful in the prevention and/or treatment of complications due to low oxygen delivery to peripheral tissues, in particular complications of SCT, more particularly diabetic complications in SCT patients or in Thalassemia.

### Pharmaceutical compositions

Dalcetrapib and dalcetrapib-thiol or a combination thereof can be administered as a pharmaceutical formulation comprising dalcetrapib and dalcetrapib-thiol or a combination thereof and a pharmaceutically acceptable carrier, diluent or excipient thereof.

Pharmaceutical compositions of the invention can contain one or more agent(s) of the invention in any form described herein. Compositions of this invention may further comprise one or more pharmaceutically acceptable additional ingredient(s), such as alum, stabilizers, antimicrobial agents, buffers, coloring agents, flavoring agents, adjuvants, and the like.

The agents of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active agents or principles, and such unit dosage forms may contain any suitable effective amount of the active ingredient commensurate with the intended dosage range to be employed. Compositions according to the invention are preferably oral.

Compositions of this invention may be liquid formulations, including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry powder for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, fructose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non-aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in out in Part 5 of Remington's "The Science and Practice of Pharmacy", 22nd Edition, 2012, University of the Sciences in Philadelphia, Lippincott Williams & Wilkins, which is incorporated herein by reference. Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, acacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or non-aqueous vehicles including, but not limited to, creams, ointments, lotions, pastes, medicated plaster, patch, or membrane.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the *stratum corneum,* and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting agents across the *stratum corneum.*

The agents of this invention can also be administered in sustained release forms or from sustained release drug delivery systems. A description of representative sustained release materials can also be found in the incorporated materials in Remington's Pharmaceutical Sciences.

*Patients*

In an embodiment, subjects according to the invention are subjects suffering from a disease or disorder characterized by inefficient transport and delivery of oxygen to peripheral tissues.

In another embodiment, subjects according to the invention are subjects suffering from or at risk from suffering from complications of SCT.

In another further embodiment, subjects according to the invention are subjects suffering from or at risk from suffering from diabetic complications of SCT or of thalassemia.

In another further embodiment, subjects according to the invention are subjects suffering from complications of SCT such as eye diseases, neurodegenerative diseases, kidney diseases, pulmonary diseases as defined herein.

In another embodiment, subjects according to the invention suffering from exaggerated response to adrenergic stimuli associated with polymorphism in Adenylyl Cyclases in particular type 9 as well as Beta adrenergic receptor type 2 polymorphism (rs1042713 Gly16Arg and rs1042714 (Gln27Glu) as described in Niesor et al., 2015, Archives of Medical Research, 46 361-371 and Niesor et al., 2015, Cardiovasc Drugs Ther., 29(6):563-572.

*Use of the invention*

According to a particular aspect, is provided a useof a dalcetrapib compound selected from dalcetrapib, dalcetrapib-thiol and a mixture thereof for the prevention and/or treatment of Sickle cell trait (SCT) or a complication thereof.

According to a further particular aspect, is provided a useof a dalcetrapib compound selected from dalcetrapib, dalcetrapib-thiol and a mixture thereof for the prevention and/or treatment of diabetes SCT complications.

According to another further particular aspect, is provided a useof a dalcetrapib compound selected from dalcetrapib, dalcetrapib-thiol and a mixture thereof for the prevention and/or treatment of SCT complication selected from eye diseases, neurodegenerative diseases, kidney diseases, and pulmonary diseases.

According to another particular aspect, the dalcetrapib compound is dalcetrapib-thiol (S-{2-([[1-(2-ethylbutyl)cyclohexyl]carbonyl]amino)phenyl}2-thiol).

According to another particular aspect, the dalcetrapib compound is to be administered in combination with at least a co-agent as defined herein or a mixture thereof and a pharmaceutically acceptable carrier, diluent or excipient thereof.

According to another aspect, compounds or compositions of the invention are allosteric modulator of hemoglobin. In particular, according to a particular embodiment, compounds or compositions of the invention have a high affinity and selective covalent binding to Cys93 of the hemoglobin β chain which improves hemoglobin function: transport and delivery of oxygen. In more particular, according to a particular embodiment, when oxygen binds to one of the Hb subunits, compounds or compositions of the invention facilitate the binding of oxygen to the other subunits. Hemoglobin's cooperativity is associated with the allosteric transition from the T-state to the R-state (T for tense and R for relaxed) (Perutz, 1989, Q. Rev. Biophys., 22:139 -236*).

According to a further aspect, compounds of the invention or combinations thereof are to be administered orally.

According to a further aspect, compounds of the invention or combinations thereof are to be administered by injection, in particular by parenteral, subcutaneous or intravenous administration.

According to another aspect, the invention provides a method of preventing and/or treating, inefficient transport and delivery of oxygen to peripheral tissues, said method comprising administering dalcetrapib or dalcetrapib-thiol or a mixture thereof, to a subject in need thereof.

Dalcetrapib or dalcetrapib-thiol or a mixture thereof or a pharmaceutical formulation thereof, can be administered to an individual prior to, simultaneously or sequentially with at least one further co-agent. Co-agents or pharmaceutical formulations thereof that are administered simultaneously with dalcetrapib or dalcetrapib-thiol, can be administered in the same or different composition(s) and by the same or different route(s) of administration.

According to one aspect, a combination of the invention advantageously prevents or decreases or treats the micro and macrovascular complications of SCT.

**EXAMPLES**

The following abbreviations refer respectively to the definitions below:
**RBC** (red blood cell).

**Example 1: Direct binding of dalcetrapib-thiol to hemoglobin and Cys93 peptide**

Dalcetrapib SellenckenChem (China) binding studies were performed on human hemoglobin or synthetic peptide containing Cys93: GTFATLSELHCDKLHVDPENFR (SEQ ID NO: 1) from Genscript according to the protocol described by Kassa et al, 2017, Metallomics, 20;9(9): 1260-1270.

**Example 2: Comparision of GSH and dalcetrapib potency to increase the R-state of hemoglobin as measured by polymerisation of HbS**

The effect of dalcetrapib on maintaining Hb in the oxygenated states thus preventing hemoglobin S (HbS) polymerization process was evaluated mixing directly HbS and dalcetrapib to obtain a 102.7 $\mu$M final solution. GSH was tested at a final concentration of 4 mM (reference) and dalcetrapib at 100 $\mu$M. Under these conditions, dalcetrapib is known to be rapidly converted into the dalcetrapib-thiol form. The proton transverse relaxation time (T2) was measured during 15 hours of experiment using a Tecmag NMR console coupled to one homogeneous permanent magnet (B0=0.095 T, v0= 4.03 MHz) to obtain a three stage sigmoidal behavior where the delay time can be determined as the time corresponding to the interception between the linear behavior of the first and second stage. The Hb samples were obtained from fresh venous whole blood donated by voluntary sickle cell patients, and immediately heparinized. Whole blood was centrifuged in a bench centrifuge at 1,500 g for 10 min, after which the plasma and buffy coat were aspirated. The packed blood cells were washed three time with buffer saline phosphate (pH 7.4; Sigma-Aldrich Company, St. Louis, MO, USA) and then lysed by freezing (11,12). After freezing, the hemolyzed sample was centrifuged (2,000 g, 10 min) to remove the stroma, 350 mL of Hb were deposited in an NMR tube for the experiment. The Proton Magnetic Resonance (PMR) T2 was determined in a relaxometer Giromag 01® (4 MHz) (Centro de Biofisica Medica, Universidad de Oriente, Santiago de Cuba, Cuba) using a Hahn pulse sequence at 36°C, with an error less than 5% *(Adolfo Fernandez García, et al., 2005, Hemoglobin, 29 (3):181-187).* The measurements were performed during 8h of spontaneous deoxygenation.

After mixing, the HbS samples containing dalcetrapib, and the reference from the same patient (HbS without dalcetrapib), were incubated during 30 min at 4°C. Then, the samples were deposited in the NMR containers at 36°C. T2 was determined using a Tecmag NMR console coupled to one homogeneous permanent magnet (B0=0.095 T, v0=4.03MHz for protons). With that purpose, the Carr Purcell Meiboon Gill experiment (CPMG) Meiboom et al., 1958, Rev. Sci. Inst., 29, 688-690 was implemented using the parameters of **Table 1:**

**Table 1**

| | |
|---|---|
| Acquisition time: 200 $\mu$s | Echo time: 1000 $\mu$s |
| Number of echoes: 1000 | Acquisition points: 8 (each echo) |
| 180 degree pulse width: 10 $\mu$s | Points ID: 8000 |
| 90 degree pulse width: 5 $\mu$s | SW: 20 000 Hz |
| Post Delay: 2500 ms | Filter: 20 000 Hz |
| Preq: 10 $\mu$s | Dwell Time: 25 $\mu$s |
| Last Delay: 12 s | Scan ID: 20 |
| Receiver gain: 200 | |

The Preq parameter is a specific delay inside the specific pulse sequence programming solution implemented in the NMR equipment used.

T2 was plotted against the time of the experiment taking the zero time as the time in which the NMR container was located under 36°C of temperature; a Boltzmann sigmoidal fit was performed. The DT (Delay Time) was determined as previously described in Lores et al., 2005, Applied Magnetic Resonance, 28. (1). 79-84 and *Adolfo Fernandez Garcia, et al., 2005, supra.,* as the time corresponding to the interception between the linear behaviors of T2 during the first and second stage of the HbS polymerization; the polymerization rate was obtained as the slope of the linear behavior representing the polymerization state and the other parameters were extracted directly from the Sigmoidal fit. Fitting of the kinetic curves to the Boltzman function and the statistical analysis was carried out using the program OriginPro™ 8 SRO v8 (OriginLab Corporation, Northampton, MA, USA). To compare the mean values of DT determined using the magnetic resonance method, the student's test was employed, t-test (a=0.01). Before the t-test, the normal distribution of the data was demonstrated and the Fisher's test, F-test (a=0.01), was used for the variances analysis. DT values of the HbS sample treated with 4 mM GSH and its own reference (HbS without GSH) are presented under **Table 2** below:

**Table 2**

| Sample | Delay Time HbS (reference) (min) | Delay Time HbS + 4mM GSH (min) | Deviation percentage from the reference (%) |
|---|---|---|---|
| 1 | 412.16 | 459.77 | **11.55** |
| 2 | 517.46 | 526.46 | 1.74 |
| 3 | 517.46 | 549.91 | **6.27** |
| 4 | 445.79 | 494.44 | **10.91** |
| 5 | 445.79 | 463.72 | **4.02** |

The HbS samples treated with GSH (4 mM) increased the HbS polymerization delay time between 4% and 11 %, compared to not treated HbS samples from the same patient. It is statistically significant considering the percentage variation of delay time in the same individual is less than 2 %. $T_2$ values of the HbS sample treated with dalcetrapib (100 $\mu$M) and its own reference (HbS without dalcetrapib) are compared in **Table 3** ($T_2$ initial values) and **Table 4** ($T_2$ final values).

**Table 3**

| Sample | $T_2$ initial values HbS reference (ms) | $T_2$ initial values HbS + dalcetrapib (ms) | Deviation from reference (%) |
|---|---|---|---|
| 1 | 90 | 110 | 22.22 |
| 2 | 90 | 108.22 | 20.24 |
| 3 | 106.71 | 140.23 | 31.41 |
| 4 | 106.71 | 119.10 | 11.61 |
| 5 | 126.82 | 151.71 | 19.62 |
| 6 | 126.82 | 151.6 | 18.48 |

**Table 4**

| Sample | $T_2$ final values HbS reference (ms) | $T_2$ final values HbS + dalcetrapib (ms) | Deviation from reference (%) |
|---|---|---|---|
| **1** | 49.59 | 68.22 | 37.57 |
| **2** | 49.59 | 68.84 | 38.83 |
| **3** | 91.16 | 112.38 | 23.28 |
| **4** | 91.16 | 99.69 | 9.35 |
| **5** | 102.43 | 122.53 | 19.63 |
| **6** | 102.43 | 122.53 | 19.63 |

The value ratios $\dfrac{T_2^{initial}}{T_2^{final}}$ and the polymerization rate values of the HbS sample treated with dalcetrapib and its own reference (HbS without dalcetrapib) are presented under **Tables 5** and 6, respectively.

**Table 5**

| Sample | $\dfrac{T_2^{initial}}{T_2^{final}}$ HbS reference | $\dfrac{T_2^{initial}}{T_2^{final}}$ HbS + dalcetrapib | Deviation from treference (%) |
|---|---|---|---|
| 1 | 1.8151 | 1.6126 | -11.16 |
| 2 | 1.8151 | 1.5720 | -13.39 |
| 3 | 1.1706 | 1.2478 | 6.59 |
| 4 | 1.1706 | 1.1948 | 2.06 |
| 5 | 1.2382 | 1.2381 | -0.005 |
| 6 | 1.2382 | 1.2263 | -0.96 |

**Table 6**

| Sample | Polymerization rate HbS reference (ms/min) | Polymerization rate HbS + dalcetrapib (ms/min) | Deviation from reference (%) |
|---|---|---|---|
| 1 | 0.2863 | 0.1549 | -45.89 |
| 2 | 0.2863 | 0.1925 | -32.76 |
| 3 | 0.131 | 0.2126 | 62.29 |
| 4 | 0.131 | 0.2011 | 53.51 |
| 5 | 0.0558 | 0.1261 | 125.98 |
| 6 | 0.0558 | 0.1121 | 100.89 |

An increased T2 initial and T2 final values was observed for all patients and the delay time with respect to the reference was obtained for the large majority of samples treated with dalcetrapib. Since in 100% of the samples incubated with dalcetrapib, both the initial value and the final value of T2 increases with respect to its value in the case of the reference (untreated sample) dalcetrapib is considered as affecting positively HbS polymerisation. The deviation of the initial/final value of T2 from its respective reference value is greater than the variation of the initial/final value of T2 in the same individual.

## Claims

1. A dalcetrapib compound selected from dalcetrapib, dalcetrapib-thiol and a combination thereof for use in the prevention and/or treatment of a disease or disorder **characterized by** inefficient transport and delivery of oxygen to peripheral tissues.

2. A use according to claim 1, wherein said disease or disorder is Sickle cell trait (SCT) or a complication thereof.

3. A use according to claim 1 or 2, diabetic SCT complications such as retinopathy, kidney disease, hypertension, neurodegenerative diseases such as Alzheimer and Parkinson diseases, 2,3 biphosphoglycerate metabolic disorders, hypoxic tumors, acute or chronic ischemia, hemorrhagic shock, anemia, cerebral and cardiovascular diseases such as hypertension, stroke, coronary artery disease, heart failure and myocardial infarction, pulmonary diseases and in particular chronic pulmonary diseases such as asthma, COPD, chronic hypoxia, altitude sickness, gangrene, anaerobic infections, eye diseases such as macular degeneration, cataract, retinal degeneration, kidney diseases, autoimmune diseases and sepsis.

4. A use according to any one of claims 2 to 3, wherein complications of SCT are diabetic complications in SCT.

5. A use according to any one of claims 2 to 3, wherein complications of SCT are selected from eye diseases, degenerative diseases, kidney diseases, and pulmonary diseases.

6. A use according to any one of claims 2 to 3, wherein complications of SCT is a pulmonary disease such as asthma or COPD.

7. A use according to claim 1, wherein said disease or disorder is diabetic complications in Thalassemia.

8. A use according to any one of claims 1 to 7, wherein the dalcetrapib compound is dalcetrapib-thiol (S-{2-([[1-(2-ethylbutyl)cyclohexyl]carbonyl]amino)phenyl}2-thiol).

9. A use according to any one of claims 1 to 8, wherein the dalcetrapib compound is to be administered at a dose of about between 300 to 900 mg per day.

10. A use according to any one of claims 1 to 8, wherein the dalcetrapib compound is to administered as single oral dose of from 1 g to about 5 g.

11. A use according to any one of claims 1 to 10, wherein the dalcetrapib compound is to be administered in combination with at least a co-agent selected from vanillin and derivatives, GBT-440 and analogues thereof.

12. A pharmaceutical composition comprising of at least one dalcetrapib compound selected from dalcetrapib, dalcetrapib-thiol and a mixture thereof and at least one co-agent selected from vanillin and derivatives, GBT-440 and analogues thereof.

13. A use according to any one of claims 1 to 10, wherein the dalcetrapib compound is to be administered in the form of a pharmaceutical composition according to claim 12.

Europäisches
Patentamt

European
Patent Office

Office européen
des brevets

**EUROPEAN SEARCH REPORT**

Application Number

EP 19 16 1933

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 275 458 A1 (HARTIS-PHARMA SARL [CH]) 31 January 2018 (2018-01-31) | 1-10,13 | INV. A61K31/167 A61P7/06 A61P43/00 |
| Y | * abstract * * paragraphs [0041] - [0043] * * claims 1,10 * | 11,12 | |
| Y | ABRAHAM D J ET AL: "VANILLIN, A POTENTIAL AGENT FOR THE TREATMENT OF SICKLE CELL ANEMIA", BLOOD, vol. 77, no. 6, 15 March 1991 (1991-03-15), pages 1334-1341, XP001080703, ISSN: 0006-4971 * abstract * | 11,12 | |
| Y | RENÉE V. GARDNER: "Sickle Cell Disease: Advances in Treatment", OCHSNER JOURNAL, vol. 18, no. 4, 13 December 2018 (2018-12-13), pages 377-389, XP055621800, ISSN: 1524-5012, DOI: 10.31486/toj.18.0076 | 11,12 | |
| A | * page 382, column 1, last paragraph - column 2, paragraph 1 * | 1-10,13 | TECHNICAL FIELDS SEARCHED (IPC) A61K A61P |
| A | GREGORY G. SCHWARTZ ET AL: "Effects of Dalcetrapib in Patients with a Recent Acute Coronary Syndrome", NEW ENGLAND JOURNAL OF MEDICINE, vol. 367, no. 22, 29 November 2012 (2012-11-29), pages 2089-2099, XP055217567, ISSN: 0028-4793, DOI: 10.1056/NEJMoa1206797 * the whole document * | 1-13 | |

-/--

The present search report has been drawn up for all claims

| Place of search | Date of completion of the search | Examiner |
|---|---|---|
| Munich | 13 September 2019 | Rodríguez-Palmero, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**

**European Patent Office**

**Office européen des brevets**

# EUROPEAN SEARCH REPORT

Application Number

EP 19 16 1933

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TAIWO R. KOTILA: "Sickle Cell Trait: A Benign State?", ACTA HAEMATOLOGICA, vol. 136, no. 3, 16 July 2016 (2016-07-16), pages 147-151, XP055621828, CH ISSN: 0001-5792, DOI: 10.1159/000446526 * the whole document * ----- | 1-13 | |

TECHNICAL FIELDS
SEARCHED      (IPC)

The present search report has been drawn up for all claims

| Place of search | Date of completion of the search | Examiner |
|---|---|---|
| Munich | 13 September 2019 | Rodríguez-Palmero, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 16 1933

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2019

| Patent document cited in search report | | Publication date | Patent family member(s) | Publication date |
|---|---|---|---|---|
| EP 3275458 | A1 | 31-01-2018 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9765017 B
- US 10047063 B
- US 9713602 B

**Non-patent literature cited in the description**

- **ACKERS et al.** *Science,* 1992, vol. 255 (5040), 54-63
- **KHAN et al.** *Biochemistry,* 2001, vol. 40, 7581-7592
- **JIA et al.** *Nature,* 1996, vol. 380, 221-226
- **ALLISON.** *Br. Med. J.,* 1954, vol. 1 (4587), 290-294
- **THOMPSON.** *Br. Med. J.,* 1963, vol. 14;2 (5371), 1534
- **LU et al.** *Am. J. Hematol.,* 2018, vol. 93, 1227-1235
- **JOHN.** *OMJ,* 2010, vol. 25, 3-8
- **NAIK et al.** *JAMA,* 2014, vol. 312 (20), 2115-2125
- **NELSON et al.** *N. Engl. J. Med.,* 2016, vol. 4; 375 (5), 435-442
- **FOLSOM et al.** *J Thromb Haemost.,* 2015, vol. 13 (1), 2-9
- **NAIK et al.** *Blood,* 2016, vol. 18 (127), 2661-2663
- **KEY et al.** *Br. J. Haematol.,* 2015, vol. 170 (1), 5-14
- **SKINNER.** *Diabetes Care,* 2018, vol. 41, 1-8
- **WEATHERALL et al.** *Nat Med,* 1996, vol. 2, 847-9
- **STRADER et al.** *Redox Biology,* 2016, vol. 8, 363-374
- **KASSA et al.** *Metallomics,* 2017, vol. 9 (9), 1260-1270
- **METCALF et al.** *ACS Med. Chem. Lett.,* 2017, vol. 8, 321-326
- **OKAMOTO et al.** *Nature,* 2000, vol. 406, 203-7
- **GUARNONE et al.** *Haematologica,* 1995, vol. 80, 426-30
- **PETERSMANN.** *Exp Clin Endocrinol Diabetes,* 2018, vol. 126 (7), 406-410
- **HARRIS.** *Nutr Metab Cardiovasc Dis.,* 2000, vol. 10 (6), 305-9
- **NGUYEN-KHOA.** *Ann Biol Clin.,* 2018, vol. 76 (4), 416-42
- **MOAT.** *Clin Chem.,* 2014, vol. 60 (2), 373-80
- **RAB et al.** *Am J Hematol.,* 2019
- Remington's "The Science and Practice of Pharmacy. University of the Sciences in Philadelphia, Lippincott Williams & Wilkins, 2012
- **NIESOR et al.** *Archives of Medical Research,* 2015, vol. 46, 361-371
- **NIESOR et al.** *Cardiovasc Drugs Ther.,* 2015, vol. 29 (6), 563-572
- **PERUTZ.** *Q. Rev. Biophys.,* 1989, vol. 22, 139-236
- **KASSA et al.** *Metallomics,* 2017, vol. 20;9 (9), 1260-1270
- **ADOLFO FERNANDEZ GARCÍA et al.** *Hemoglobin,* 2005, vol. 29 (3), 181-187
- **MEIBOOM et al.** Carr Purcell Meiboon Gill experiment (CPMG). *Rev. Sci. Inst.,* 1958, vol. 29, 688-690
- **LORES et al.** *Applied Magnetic Resonance,* 2005, vol. 28 (1), 79-84